# EUROPÄISCHE PATENTSCHRIFT

(11) **EP 1 861 343 B1**
(45) Veröffentlichungstag und Bekanntmachung des Hinweises auf die Patenterteilung: **04.03.2009**
(21) Anmeldenummer: 06723260.3
(22) Anmeldetag: 07.03.2006
(51) Int. Cl.: C07B 37/04, C07C 17/26, C07C 67/30, C07C 25/13, C07C 69/76, C07F 5/02

(54) **VERFAHREN ZUR HERSTELLUNG VON ALKYLSUBSTITUIERTEN AROMATEN UND HETEROAROMATEN DURCH KREUZKUPPLUNG VON ALKYLBORONSÄUREN MIT ARYL- ODER HETEROARYLHALOGENIDEN ODER -SULFONATEN UNTER PD-KATALYSE IN GEGENWART EINES LIGANDEN**
METHOD FOR PRODUCING ALKYL-SUBSTITUTED AROMATIC AND HETEROAROMATIC COMPOUNDS BY CROSS-COUPLING ALKYL BORONIC ACIDS WITH ARYL- OR HETEROARYL-HALOGENIDES OR SULFONATES UNDER PD CATALYSIS IN THE PRESENCE OF A LIGAND
PROCEDE POUR PRODUIRE DES COMPOSES AROMATIQUES ET HETEROAROMATIQUES A SUBSTITUTION ALKYLE PAR COUPLAGE CROISE D'ACIDES ALKYLBORONIQUES AVEC DES HALOGENURES D'ARYLE OU D'HETEROARYLE OU DES ARYLSULFONATES OU HETEROARYLSULFONATES AVEC CATALYSE PD EN PRESENCE D'UN LIGAND

(30) Priorität: 16.03.2005 DE 102005012005
(43) Veröffentlichungstag der Anmeldung: 05.12.2007
(73) Patentinhaber: Archimica GmbH, 65929 Frankfurt am Main (DE)
(72) Erfinder: SCHERER, Stefan, 64347 Griesheim (DE); MEUDT, Andreas, 65719 Hofheim (DE); NERDINGER, Sven, 83088 Kiefersfelden (DE); LEHNEMANN, Bernd, 60326 Frankfurt (DE); JAGUSCH, Thomas, 47809 Krefeld (DE); SNIECKUS, Victor, Kingston, ON, K7L 1R2 (CA)
(74) Vertreter: Schweitzer, Klaus
(86) Internationale Anmeldenummer: PCT/EP2006/002061
(87) Internationale Veröffentlichungsnummer: WO 2006/097221

(56) Entgegenhaltungen:
- KONDOLFF I ET AL: "Tetraphosphine/palladium catalysed Suzuki cross-coupling reactions of aryl halides with alkylboronic acids" TETRAHEDRON, ELSEVIER SCIENCE PUBLISHERS, AMSTERDAM, NL, Bd. 60, Nr. 17, 19. April 2004 (2004-04-19), Seiten 3813-3818, XP004501252 ISSN: 0040-4020
- NORIYASU KATAOKA, QUINETTA SHELBY, JAMES P. STAMBULI, JOHN F. HARTWIG: "Air Stable, Sterically Hindered Ferrcenyl Dialkylphosphines for Palladium-Catalyzed C-C, C-N, and C-O Bond-Forming Cross-Coupling" JOURNAL OF ORGANIC CHEMISTRY, Bd. 67, 2002, Seiten 5553-5566, XP002400233
- MOLANDER G A ET AL: "Cross-coupling reactions of primary alkylboronic acids with aryl triflates and aryl halides" TETRAHEDRON, ELSEVIER SCIENCE PUBLISHERS, AMSTERDAM, NL, Bd. 58, Nr. 8, 18. Februar 2002 (2002-02-18), Seiten 1465-1470, XP004336380 ISSN: 0040-4020
- WRIGHT S W ET AL: "Fluoride-Mediated Boronic Acid coupling reactions" JOURNAL OF ORGANIC CHEMISTRY, AMERICAN CHEMICAL SOCIETY. EASTON, US, Bd. 59, 1994, Seiten 6095-6097, XP002206523 ISSN: 0022-3263
- ORTIZ R ET AL: "Tandem intramolecular carbolithiation-transmetallation: from lithium to copper or boron chemistry" TETRAHEDRON, ELSEVIER SCIENCE PUBLISHERS, AMSTERDAM, NL, Bd. 61, Nr. 7, 14. Februar 2005 (2005-02-14), Seiten 1699-1707, XP004744664 ISSN: 0040-4020
- HARVEY J E ET AL: "Synthesis of non-proteinogenic phenylalanine analogues by Suzuki cross-coupling of a serine-derived alkyl boronic acid" TETRAHEDRON LETTERS, ELSEVIER, AMSTERDAM, NL, Bd. 45, Nr. 11, 8. März 2004 (2004-03-08), Seiten 2467-2471, XP004491474 ISSN: 0040-4039
- CHUANSHENG N ET AL: "Synthesis of 3-Aminopyridine-2-carboxaldehyde Thiosemicarbazone (3-AP)" TETRAHEDRON, ELSEVIER SCIENCE PUBLISHERS, AMSTERDAM, NL, Bd. 54, Nr. 23, 4. Juni 1998 (1998-06-04), Seiten 6311-6318, XP004119579 ISSN: 0040-4020
- ZOU G ET AL: "Ag(I)-promoted Suzuki-Miyaura cross-couplings of n-alkylboronic acids" TETRAHEDRON LETTERS, ELSEVIER, AMSTERDAM, NL, Bd. 42, Nr. 41, 8. Oktober 2001 (2001-10-08), Seiten 7213-7215, XP004304969 ISSN: 0040-4039
- HERMANN W A ET AL: "Synthesis, structure and catalytic aplication of Pd(II)complexes bearing N-heterocyclic carbenes and phosphines" JOURNAL OF ORGANOMETALLIC CHEMISTRY, ELSEVIER-SEQUOIA S.A. LAUSANNE, CH, Bd. 617-618, 15. Januar 2001 (2001-01-15), Seiten 616-628, XP002178611 ISSN: 0022-328X

## Beschreibung

Alkylsubstituierte Aromaten und Heteroaromaten, vor allem mit funktionellen Gruppen in der Alkylkette, sind bedeutsame und äußerst vielseitig verwendbare Zwischenprodukte in der organischen Synthese. Die Bedeutung in der modernen organischen Synthese wird nur durch Limitierungen der Zugänglichkeit dieser Verbindungsklasse eingeschränkt. Standardverfahren zur Herstellung von alkylsubstituierten Aromaten und auch Heteroaromaten ist die *Friedel-Crafts-*Alkylierung, wobei die Reaktion meist nicht regioselektiv abläuft. Ferner tolerieren die Reaktionsbedingungen, die in der Regel sehr drastisch sind, selten funktionelle Gruppen und reaktive Heteroaromaten und sind nicht oder nur sehr schlecht anwendbar auf elektronenarme Aromaten und schwierig zu kontrollieren,

In der modernen organischen Synthese nimmt die Bedeutung chemo-, regio- und stereoselektiver Reagentien explosionsartig zu. Will man beispielsweise in einem substituierten Aromaten, dessen Substituenten eine elektrophile Substitution unterschiedlich dirigieren, eine Alkylgruppe in eine bestimmten Position einführen, können unselektive Methoden wie die *Friedel-Crafts*-Alkylierung keine Anwendung mehr finden.

Es wäre daher sehr wünschenswert, ein Verfahren zu haben, das Alkylboronsäuren und Halogenaromaten bzw. Halogenheteroaromaten in die entsprechenden alkylsubstiuierten Aromaten bzw. Heteroaromaten überführen kann, dabei gleichzeitig sehr hohe Ausbeuten erzielt und mit sehr geringen Katalysatormengen auskommt und zusätzlich in wirtschaftlich nutzbaren Verfahren einsetzbar ist.

Aus KONDOLFFI ET AL ("Tetraphosphine/palladium catalysed Suzuki cross-coupling reactions of aryl halides with alkylboronic acids". TETRAHEDRON, ELSEVIER SCIENCE PUBUSHERS, AMSTERDAM, NL, Bd. 60, Nr, 17, 19. April 2004 (2004-04-19), Seiten 3813-3818, XP004501252 ISSN: 0040-4020), NORIYASU KATAOKA, QUINETTA SHELBY, JAMES P. STAMBULI, JOHN F. HARTWIG ("Air Stable, Sterically Hindered Ferrcenyl Dialkylphosphines for Palladium-Catalyzed C-C, C-N, and C-O Bond-Forming Cross-Coupling" JOURNAL OF ORGANIC CHEMISTRY, Bd, 67, 2002, Seiten 5553-5566, XP002400233),
MOLANDER G A ET AL ("Cross-coupling reactions of primary alkylboronic acids with aryl triflates and aryl halides" TETRAHEDRON, ELSEVIER SCIENCE PUBLISHERS, AMSTERDAM, NL, Bd. 58, Nr. 8,18. Februar 2002 (2002-02-18), Seiten 1465-1470, XP004336380 ISSN: 0040-4020),
HARVEY J E ET AL ("Synthesis of non-proteinogenic Phenylalanine analogues by Suzuki cross-coupling of a serine-derived alkyl boronic acid" TETRAHEDRON LEITERS, ELSEVIER, AMSTERDAM, NL, Bd. 45, Nr, 11, 8. März 2004 (2004-03-08), Seiten 2467-2479, XP004491474 ISSN: 0040-4039) und
CHUANSHENG N ET AL ("synthesis of 3-Aminopyridine-2-carboxaldehyde Thiosemicarbazone (3-AP)" TETRAHEDRON. ELSEVIER SCIENCE PUBLISHERS, AMSTERDAM, NL, Bd. 54, Nr. 23, 4. Juni 1998 (1998-06-04). Seiten 6311-6318, XP004119579 ISSN: 0040-4020)
ist die Herstellung von alkylsubstituierten Aromaten und Heteroaromaten im Zuge einer Kreuzkupplung von Alkylboronsäuren mit Arylhalogeniden bzw. Heteroarylhalogeniden vermittels Palladiumkatalyse mit einem phosphorhaltigen Liganden bekannt.

Die bisher veröffentlichten Syntheseverfahren lösen das o.g. subjektive Problem jedoch nicht und zeigen viele Nachteile, wie anhand von einigen Beispielen demonstriert werden soll:
- aufwendige bzw. schwierige Ligandensynthesen (z.B. M. Santelli et al., Tetrahedron 2004, 60, 3813-3818*;* Hartwig et al. J. Org. Chem. 2002, 67, 5533-5566)
- Verwendung von großen Palladiummengen, bis zu 30 mol % (z.B. J. Med. Chem. 2001, 44, 3302-3310; Najera et al., J. Organomet. Chemistry 2002, 663, 46-57)
- niedrige Ausbeuten (z.B. Wright et al., J. Org. Chem 1994, 59, 6095-6097; Percy et al, J. Chem. Soc., Perkin Trans. 12000,2591-2599)
- erfolglos bei subst. Alkylboronsäuren (z.B. Najera et al., J. Organomet. Chemistry 2002, 663, 46-57)
- sehr geringen TON (z.B. Bedford et al. Chem. Eur. J. 2003, 9, 3216-3227)
- nicht praktikable Bedingungen, wie z.B. die Verwendung von Mikrowellen (z.B. Kabalka et al., Synthesis 2003, 217-222)
- hoher Überschuss von teuren Alkylboronsäuren nötig (z.B. Bedford et al. Chem. Eur. J. 2003, 9, 321.6-3227)

Das vorliegende Verfahren löst diese Probleme und betrifft ein Verfahren zur Herstellung von alkylsubstituierten Aromaten und Heteroaromaten (III) durch Kreuzkupplung von Alkylböronsäuren und ihren Derivaten (II) mit Arylhalogeniden bzw. Heteroarylhalogeniden (I) unter Katalyse durch Obergangsmetallverbindungen in Gegenwart von leicht zugänglichen bzw. handelsüblichen Liganden und einer Bronsted-Base in einem Lösungsmittel, wobei mit geringen Katalysatorbeladungen hohe Ausbeuten erzielt werden (Gleichung 1).

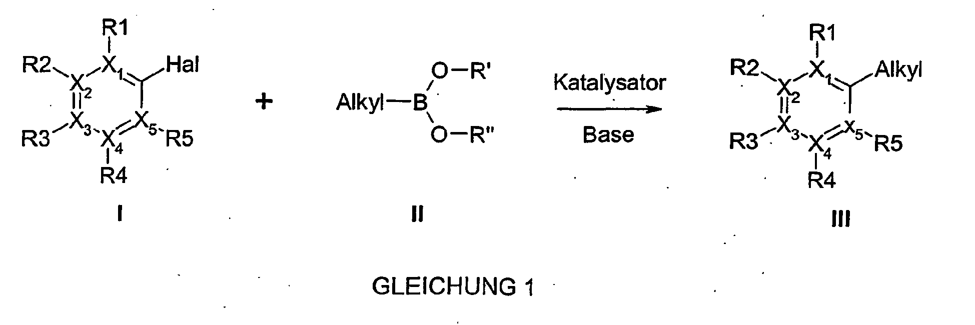

Dabei steht
- Hal: für Chlor, Brom, Iod, Trifluormethansulfonat, Nonafluortrimethylmethansulfonat, Methansulfonat, 4-Toluolsulfonat, Benzolsulfonat, 2-Naphtalensulfonat, 3-Nitrobenzolsulfonat, 4-Nitrobenzolsulfonat 4-Chlorbenzolsulfonat oder 2,4,6-Triisopropylbenzolsulfonat,
- X₁₋₅: stehen unabhängig voneinander entweder für Kohlenstoff oder
- X₁R₁: steht für Stickstoff, oder jeweils zwei benachbarte über eine formale Doppelbindung verbundene XᵢRᵢ stehen gemeinsam für O (Furane), S (Thiophene), NH oder NRᵢ (Pyrrole).

Bevorzugte Verbindungen der Formel (III), die nach dem erfindungsgemäßen Verfahren umgesetzt werden können, sind z.B. Benzole, Pyridine, Pyrimidine, Pyrazine, Pyridazine, Furane, Thiophene, Pyrrole, beliebig N-substituierte Pyrrole oder Naphthaline, Chinoline, Indole, Benxofurane usw.

Die Reste R₁₋₅ stehen für Substituenten aus der Gruppe {Wasserstoff, Methyl, primäre, sekundäre oder tertiane, cyclische oder acyclische Alkylreste mit 2 bis 20 C-Atomen, bei denen gegebenenfalls ein oder mehrere Wasserstoffatome durch Fluor oder Chlor oder Brom ersetzt sind, z.B. CF₃, substituierte cyclische oder acyclische Alkylgruppen, Hydroxy, Alkoxy, Amino, Alkylamino, Dialkylamin, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Fluor oder Chlor, Nitro, Cyano, Aryl- oder Alkylsulfon, Aryl- oder Alkylsulfonyl},
oder es können jeweils zwei benachbarte Reste R₁₋₅
zusammen einem aromatischen, heteroaromatischen oder aliphatischen ankondensierten Ring entsprechen.

Alkyl kann für beliebige lineare, verzweigte oder cyclische Alkylreste mit 1 bis 40, vorzugsweise 1-20, insbesondere 1-8 C-Atomen stehen, bei denen gegebenenfalls ein oder mehrere Wasserstoffatome durch Fremdatome oder Gruppen ersetzt sind, die unter den Reaktionsbedingungen inert sind, z,B, Fluor, Chlor oder Brom, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphina, Dialkylphosphino, Alkylarylphosphino, gegebenenfalls substituiertes Aminocarbonyl, CO₂⁻ , Alkyl- oder Aryloxycarbonyl, Hydroxyalkyl, Alkoxyalkyl, Fluor oder Chlor, Nitro, Cyano, Aryl- oder Alkylsulfon.

R' und R" können unabhängig voneinander für gleiche oder unterschiedliche Reste aus der Gruppe {Wasserstoff, Methyl, lineares, verzweigtes oder cyclisches gegebenenfalls substituiertes Alkyl, substituiertes oder unsubstituiertes Phenyl} stehen oder zusammen einen Ring bilden und aus der Gruppe {gegebenenfalls substituiertes Alkylen, verzweigtes Alkylen, cyclisches Alkylen, oder gegebenenfalls substituiertes Azaalkylen} stammen.

Typische Beispiele für die Verbindung II sind damit Methan-, Ethan-, 1-Methylethan-, Propan-, 1-Methylpropan-, 2-Methylpropan-, 1,1-Dimethylethan-, Butan- und Pentanboronsäure, Cyclopropan-, Cyclobutan-, Cyclopentan-, Cyclohexanboronsäure etc. sowie ihre Methyl-, Ethyl-, Propyl-, Isopropyl-, Cyclohexylester etc. sowie ihre Ethylenglycol-, Pinacol- und Neopentylglycolester etc. und ihre Addukte mit Diethanolamin, N-Methyl- oder N-Phenyldiethanolamin.

Als Katalysator wird erfindungsgemäß ein Salz, ein Komplex oder eine metallorganische Verbindung eines Metalls der Gruppe {Mn, Fe, Co, Ni, Cu, Rh, Pd, Ir, Pt} verwendet, bevorzugt Palladium oder Nickel. Der Katalysator kann in fertiger Form zugesetzt werden oder sich in situ bilden, z.B. aus einem Präkatalysator durch Reduktion oder Hydrolyse oder aus einem Metallsalz und zugesetztem Liganden durch Komplexbildung. Der Katalysator wird in Kombination mit einem oder mehreren phosphorhaltigen Liganden eingesetzt. Das Metall kann in beliebiger Oxidationsstufe eingesetzt werden. Erfindungsgemäß wird es im Verhältnis zum Reaktanden I in Mengen von 0,001 Mol-% bis 100 Mol-%, bevorzugt zwischen 0,01 und 10 Mol-%, besonders bevorzugt zwischen 0.01 und 1 Mol-% eingesetzt.

Eingesetzt als Katalysatoren werden Liganden der Struktur 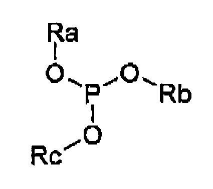 in Verbindung mit Palladium oder Nickel,
Ra, Rb und Rc
stehen dabei unabhängig voneinander für jeweils einen geradkettigen, verzweigten oder cyclischen Alkylrest oder Arylrest, der gegebenenfalls Substituenten aus der Gruppe
{geradkettiges oder verzweigtes Alkyl, insbesondere Methyl oder iso-Propyl, Cycloalkyl, Aryl, Fluor, Chlor} trägt,
oder zwei dieser Reste zusammen einen Ring formen können und der Gruppe {gegebenenfalls substituiertes Alkylen, gegebenenfalls substituiertes ortho-Arylen} entstammen oder drei dieser Reste einen Bicyclus formen können und der Gruppe {gegebenenfalls substituierte Trialkylole} entstammen.

Ebenfalls eingesetzt werden kann ein Ligand der Struktur 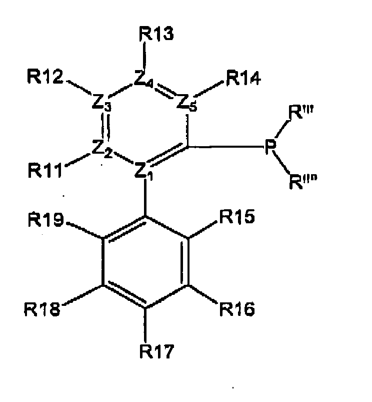 in Verbindung mit Palladium oder Nickel als Katalysator, wobei
- Z₁: für Kohlenstoff oder Stickstoff steht,
- Z₂₋₅: unabhängig voneinander entweder für Kohlenstoff steht,
- Z_{I}R_{I}: für Stickstoff steht, oder jeweils zwei benachbarte über eine formale Doppelbindung verbundene X_{I}R_{I} mit i=2,3,4,5 gemeinsam für O (Furane), S (Thiophene), NH oder NRᵢ (Pyrrole) steht,
die Reste
R₁₁₋₁₉ für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl} steht oder jeweils zwei benachbarte Reste
R₁₁₋₁₄ zusammen für einem aromatischen, heteroaromatischen oder aliphatischen ankondensierten Ring stehen,
R"' und R""unabhängig voneinander für gleiche oder unterschiedliche Reste aus der Gruppe
{Wasserstoff, Methyl, lineares, verzweigtes oder cyclisches Alkyl, Phenyl} stehen
oder zusammen einen Ring bilden und für ein verbrückendes Strukturelement aus der Gruppe
{Alkylen, verzweigtes Alkylen, cyclisches Alkylen}
oder unabhängig voneinander für einen oder zwei polycyclische Reste, wie z.B. Norbornyl- oder Adamentyl-, stehen.

Ebenfalls können Komplexe eines sekundären Phosphans in Verbindung mit einem Palladacyclus als Katalysator der Struktur 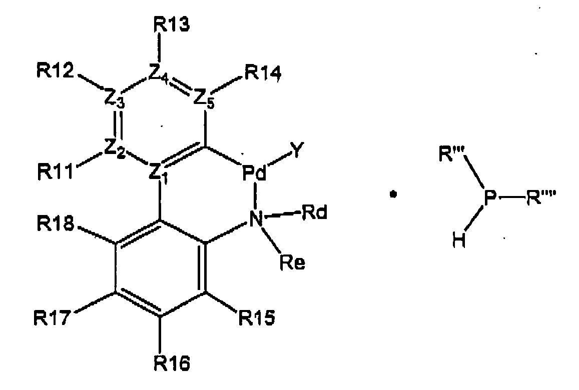 wobei die Symbole
- Z₁₋₅, R₁₁₋₁₈, R "' und R "": die vorstehend genannte Bedeutung haben und
- Y: für einen Rest aus der Gruppe {Halogenid, Pseudohalogenid, Alkylcarboxylat, Trifluoracetat, Nitrat, Nitrit} steht und
- R_{d} und Rₑ: unabhängig voneinander für gleiche oder unterschiedliche Substituenten aus der Gruppe {Wasserstoff, Methyl, primäres, sekundäres oder tertiäres gegebenenfalls substituiertes Alkyl oder Aryl} stehen oder zusammen einen Ring bilden und aus der Gruppe {gegebenenfalls substituiertes Alkylen, Oxaalkylen, Thiaalkylen, Azaalkylen} entstammen.

Geeignete Katalysatoren oder Präkatalysatoren sind erfindungsgemäß beispielsweise bestimmte Palladacyclen und ihre Phosphankomplexe (z.B. IV (Solvias SK-CC01-A)) und Komplexe von Palladium mit Biarylphosphanen, die z.T. sehr einfach und wirtschaftlich zugänglich sind (z.B. V und VI, zur Herstellung vgl. Regnat et al., EP 0 795 559) (ABBILDUNG I). Besonders hervorzuheben ist die Eignung der sehr preiswerten Trialkylphosphite, z.B. Trilsopropylphospit VII, als Liganden in Verbindung mit Palladium- oder Nickelsalzen.

Mit Katalysatoren auf Basis von Ferrocenylphosphin-Übergangsmetall-Komplexen oder sterisch gehinderten Trialkylphosphin-Übergangsmetall-Komplexen werden oft ebenfalls vollständige Umsätze der Ausgangsmaterialien erreicht.

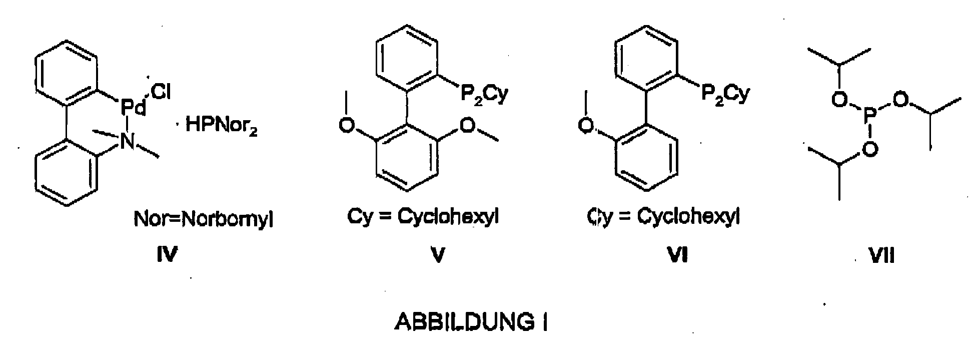

Der Zusatz von Brønsted-Basen zum Reaktionsgemisch ist notwendig, um akzeptable Reaktionsgeschwindigkeiten zu erzielen. Als Basen gut geeignet sind Hydroxide, Amine und Alkoholate und Fluoride der Alkali- und Erdalkalimetalle, Carbonate, Hydrogencarbonate und Phosphate der Alkalimetalle und ihre Gemische. Besonders geeignet sind die Basen der Gruppe {Natriumhydroxid, Kaliumhydroxid, Natriumcarbonat, Kaliumcarbonat, Cäsiumcarbonat, Natriumbicarbonat, Kaliumbicarbonat, Casiumbicarbonat, Kaliumphosphat}. Dabei wird üblicherweise mindestens die Stoffmenge an Base eingesetzt, die der Stoffmenge der Boronsäure II entspricht, zumeist werden zwischen 1.0 und 6 Äquivalente, vorzugsweise 2 bis 5 Äquivalente an Base bezogen auf die Boronsäure II eingesetzt.

Die Reaktion wird in einem geeigneten Lösungsmittel oder ein- oder mehrphasigen Lösungsmittelgemisch durchgeführt, das ein hinreichendes Lösevermögen für alle beteiligten Reaktanden hat. Gut geeignet sind offenkettige und cyclische Ether und Diether, einfache oder mehrfache Alkohole, gegebenenfalls substituierte Aromaten, Wasser, gegebenenfalls substituierte Amide, Dimethylsulfoxid, N-Methylpyrrolidon und gegebenenfalls substituierte Harnstoffe sowie Ester. Besonders bevorzugt werden ein oder Gemische mehrerer Lösungsmittel der Gruppe {Wasser, Tetrahydrofuran, 1,4-Dioxan, Methanol, Ethanol, Isopropanol, Propanol, Butanol, Ethylenglykol, Toluol, Xylol, Dimethylformamid, Dimethylacetamid} eingesetzt.

Die Reaktion kann bei Temperaturen zwischen Raumtemperatur und dem Siedepunkt des verwendeten Lösungsmittels beim verwendeten Druck durchgeführt werden. Um eine schnellere Reaktion zu erzielen, ist die Durchführung bei erhöhten Temperaturen im Bereich zwischen 20 und 240°C bevorzugt. Besonders bevorzugt ist der Temperaturbereich von 60 bis 150°C.

Die Konzentration der Reaktanden kann in weiten Bereichen variiert werden. Zweckmäßigerweise wird die Reaktion in einer möglichst hohen Konzentration durchführt, wobei die Löslichkeiten der Reaktionspartner und Reagentien im jeweiligen Reaktionsmedium beachtet werden müssen. Bevorzugt wird die Reaktion im Bereich zwischen 0.05 und 5 mol/l bezogen auf den im Unterschuss vorliegenden Reaktanden durchgeführt.

Boronsäure (II) und aromatischer bzw. heteroaromatischer Reaktionspartner (I) können in Molverhältnissen von 10:1 bis 1:10 eingesetzt werden, bevorzugt sind Verhältnisse von 3:1 bis 1:3 und besonders bevorzugt sind Verhältnisse von 1.2:1 1 bis 1:1.2.

In einer der bevorzugten Ausführungsformen werden alle Materialien vorgelegt und das Gemisch unter Rühren auf Reaktionstemperatur erhitzt. In einer weiteren bevorzugten Ausführungsform, die sich besonders für die Anwendung im großen Maßstab eignet, wird die Boronsäure und gegebenenfalls weitere Reaktanden während der Reaktion zum Reaktionsgemisch dosiert.

Die Aufarbeitung erfolgt üblicherweise wässrig unter Abtrennung der wässrigen Phase, die die anorganischen Bestandteile sowie gegebenenfalls überschüssige Boronsäure aufnimmt, wobei das Produkt in der organischen Phase verbleibt, wenn nicht vorhandene saure Funktionsgruppen zu einem abweichenden Phasenverhalten führen. Gegebenenfalls können ionische Flüssigkeiten zur Abtrennung der polareren Bestandteile eingesetzt werden. Das Produkt wird bevorzugt durch Fällung aus der organischen Phase isoliert, z.B. durch Einengen oder durch Zusatz von Fällungsmitteln. Häufig ist eine zusätzliche Reinigung z.B. durch Umkristallisation oder Chromatographie unnötig. Die isolierten Ausbeuten liegen meistens im Bereich von 75 bis 100 %, vorzugsweise im Bereich > 85 % bis 100 %, insbesondere > 92 % bis 100 %.

Das erfindungsgemäße Verfahren eröffnet eine sehr ökonomische Methode, um Alkylaromaten und Alkylheteroaromaten ausgehend von den entsprechenden Alkylboronsäuren oder ihren Derivaten und den entsprechenden Aryl- oder Heteroarylhalogeniden oder -sulfonaten herzustellen. Auf diese Weise sind damit auch erstmals Substrate zugänglich, bei denen "gewöhnliche" Suzuki-Kupplungen versagen, weil sowohl der Aryl- als auch der Alkylrest gegenüber Grignard- oder Organolithium-Zwischenstufen nicht stabile funktionelle Gruppen enthalten (Beispiel 5).

Das erfindungsgemäße Verfahren soll durch die nachfolgenden Beispiele erläutert werden, ohne die Erfindung darauf zu beschränken:

### Beispiel 1:

### Suzuki-Kupplung von Brombenzol mit n-Butylboronsäure unter Katalyse durch 2'-(Dimethylamino)-2-biphenylyl-palladium(II)-chlorid-Dinorbornylphosphin-Komplex (SK-CC01-A)

Unter Luftausschluss wurden 0.157 g Brombenzol (1.0 mmol), 0.152 g Butylboronsäure (1.5 mmol), 5.6 mg SK-CC01-A (0.01 mmol, 1 Mol-%) und 1 ml 3N Natronlauge (3 mmol) in 5 ml Dioxan unter Rühren auf 105 °C erhitzt, bis der Umsatz (It. GC) vollständig war. Man ließ abkühlen, extrahierte das Reaktionsgemisch mit 4 ml Wasser und trennte die organische Phase ab. Nach Filtration über Kieselgel (Laufmittel Ethylacetat) erhielt man 0.125 g (0.93 mmol, 93 %) n-Butylbenzol.

Beispiel 2: wie Beispiel 1, anstelle der Natronlauge wurden 637 mg (3 mmol) wasserfreies Kaliumphosphat eingesetzt. Als Lösungsmittel wurden anstelle von Dioxan 5 ml eines Gemisches aus Dimethylacetamid, Toluol und Tetrahydrofuran (1:1:1) eingesetzt. Die Ausbeute betrug 94 %.

Beispiel 3: wie Beispiel 1, anstelle der Natronlauge wurden 318 mg Natriumcarbonat (3 mmol) und 33 mg Cäsiumcarbonat (0.3 mmol) eingesetzt. Als Lösungsmittel wurden 5 ml Toluol benutzt. Die Ausbeute betrug 96 %.

Beispiel 4: wie Beispiel 1, das Lösungsmittel Dioxan wurde durch 5 ml eines Gemisches aus Toluol und Isopropanol (1:1) ersetzt. Man erhielt 94 % Butylbenzol.

### Beispiel 5:

### Suzuki-Kupplung von 4-Chlorbenzoesäureethylester mit 2-(Ethoxycarbonyl)ethylboronsäure unter Katalyse durch Dicyclohexyl-(6'-methoxy-biphenyl-2-yl)-phosphan-Palladium-Komplex

Unter Luftausschluss wurden 0.185 g 4-Chlorbenzoesäureethylester (1.0 mmol), 0.175 g 2-(Ethoxycarbonyl)ethylboronsäure (1.2 mmol), 38 mg Dicyclohexyl-(6'-methoxybiphenyl-2-yl)-phosphan (0.1 mmol, 10 Mol-%), 11 mg Palladium(II)-acetat (0.05 mmol, 5 Mol-%) und 1 ml 3N Natronlauge (3 mmol) in 5 ml Isopropanol unter Rühren auf 105 °C erhitzt, bis der Umsatz (lt. GC) vollständig war. Man ließ abkühlen, extrahierte das Reaktionsgemisch mit 4 ml Wasser und trennte die organische Phase ab. Nach Filtration über Kieselgel (Laufmittel Ethylacetat) erhielt man 0.225 g (0.90 mmol, 90%).

Beispiel 6: wie Beispiel 5, anstelle von Natronlauge wurden als Base 637 mg (3 mmol) wasserfreies Kaliumphosphat eingesetzt und als Lösungsmittel ein 1:1:1-Gemisch aus Tetrahydrofuran/Isopropanol/Toluol. Die Ausbeute betrug 88 %.

Beispiel 7: wie Beispiel 5, anstelle der Natronlauge wurden 318 mg Natriumcarbonat (3 mmol) und 33 mg Cäsiumcarbonat (0.3 mmol) eingesetzt. Als Lösungsmittel wurden 5 ml Toluol benutzt. Die Ausbeute betrug 92 %.

### Beispiel 8:

### Süzuki-Kupplung von 3-Brompyridin mit 3,3-(Diethoxy)propanboronsäure unter Katalyse durch Dicyclohexyl-(6',2'-dimethoxy-biphenyl-2-yl)-phosphan-Palladium-Komplex.

Unter Luftausschluss wurden 0.158 g 3-Brompyridin (1.0 mmol), 0.211 g 3,3-(Diethoxy)propanboronsäure (1.2 mmol), 41 mg Dicyclohexyl-(6',2'-dimethoxy-biphenyl-2-yl)-phosphan (0.1 mmol, 10 Mol-%), 11 mg Palladium(II)-acetat (0.05 mmol, 5 Mol-%) und 637 mg (3 mmol) wasserfreies Kaliumphosphat in 5 ml eines 1:1:1-Gemisch Dioxan/Tetrahydrofuran/Toluol unter Rühren auf 105°C erhitzt, bis der Umsatz (It. GC) vollständig war. Man ließ abkühlen, extrahierte das Reaktionsgemisch mit 8 ml 2N NaOH. und trennte die organische Phase ab. Nach Filtration über Kieselgel (Laufmittel Ethylacetat mit 1 % Triethylamin) erhielt man 0.195 g (0.93 mmol, 93 %) 3-(3-Pyridyl)-propionaldehyd-diethylacetal.

Beispiel 9: wie Beispiel 8, anstelle des Kaliumphosphats wurden 318 mg Natriumcarbonat (3 mmol) und 33 mg Cäsiumcarbonat (0.3 mmol) eingesetzt. Als Lösungsmittel wurden 5 ml Dioxan benutzt. Die Ausbeute betrug 87 %.

Beispiel 10: wie Beispiel 8, statt Kaliumphosphat wurde als Base 1 ml 3N aq. NaOH (3 mmol) benutzt. Als Lösungsmittel wurden 5 ml Isopropanol eingesetzt. Man erhielt 84 % Ausbeute.

Beispiel 11: wie Beispiel 8, als Lösungsmittel wurden 5 ml eines Gemisches von Tetrahydrofuran mit Wasser im Verhältnis 9:1 eingesetzt. Dabei betrug die Ausbeute 88%.

Beispiel 12: wie Beispiel 8, als Base wurde 1 ml 3N Natronlauge (3 mmol) und als Lösungsmittel 5 ml eines Gemisches von Tetrahydrofuran/Wasser/Toluol im Verhältnis 19:1:20 eingesetzt. Hierbei wurden 76 % Produkt isoliert.

Beispiel 13: wie Beispiel 12, als Base wurden 318 mg Natriumcarbonat (3 mmol) und 33 mg Cäsiumcarbonat (0.3 mmol) eingesetzt. Man erhielt 84 % Ausbeute.

### Beispiel 14:

### Suzuki-Kupplung von 4-Brombenzotrifluorid mit Butanboronsäure unter Katalyse durch Triisopropylphosphit-Palladium-Komplex

Unter Luftausschluss wurden 0.225 g 4-Brombenzotrifluorid (1.0 mmol), 0.122 g Butanboronsäure (1.2 mmol), 21 mg Trüsopropylphosphit (0.1 mmol, 10 Mol-%), 11 mg Palladium(11)-acetat (0.05 mmol, 5 Mol-%) und 1 ml 3N Natronlauge (3 mmol) in 5 ml eines 19:1:20-Gemisches Tetrahydrofuran/Wasser/Isopropanol unter Rühren auf 105°C erhitzt, bis der Umsatz (It. GC) vollständig war. Man ließ abkühlen, extrahierte das Reaktionsgemisch mit 4 ml 2N NaOH und trennte die organische Phase ab. Nach Filtration über Kieselgel (Laufmittel Ethylacetat) erhielt man 0.186 g (0.92 mmol, 92 %) 4-Butyltrifluormethylbenzol.

Beispiel 15: wie Beispiel 14, als Base wurden statt Natronlauge 637 mg (3 mmol) wasserfreies Kaliumphosphat und als Lösungsmittel Dioxan eingesetzt. Die Ausbeute betrug 92 %.

Beispiel 16: wie Beispiel 15, anstelle von Palladiumacetat wurden 6.5 mg (0.05 mmol) wasserfreies Nickel(II)-chlorid eingesetzt. Man erhielt 86 % Produkt.

## Patentansprüche

1. Verfahren zur Herstellung von alkylsubstituierten Aromaten und Heteroaromaten III durch Kreuzkupplung von Alkylboronsäuren II mit Aryl- oder Heteroarylhalogeniden oder Aryl- oder Heteroarylsulfonaten I, in Gegenwart eines Katalysators und einer Brønsted-Base in einem Lösungsmittel oder Lösungsmittelgemisch 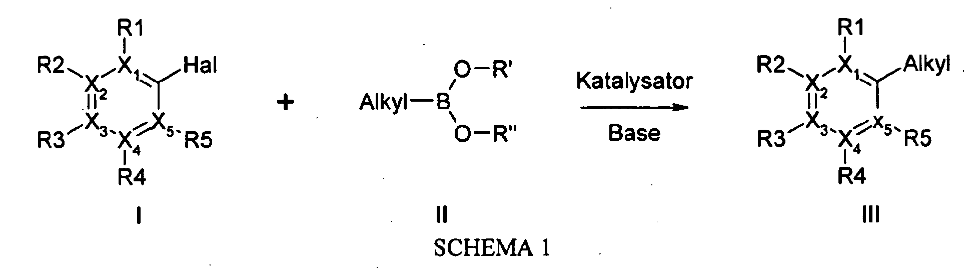 wobei
Hal für Chlor, Brom, Iod, Trifluormethansulfonat, Nonafluortrimethyl-methansulfonat, Methansulfonat, 4-Toluolsulfonat, Benzolsulfonat, 2-Naphtalensulfonat, 3-Nitrobenzolsulfonat, 4-Nitrobenzolsulfonat, 4-Chlorbenzolsulfonat oder 2,4,6-Triisopropylbenzolsulfonat steht,
X₁₋₅ unabhängig voneinander entweder für Kohlenstoff stehen,
XᵢRᵢ für Stickstoff steht, oder jeweils zwei benachbarte über eine formale Doppelbindung verbundene XᵢRᵢ gemeinsam für O (Furane), S (Thiophene), NH oder NRᵢ (Pyrrole) stehen,
die Reste
R₁₋₅ für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Fluor oder Chlor, Nitro, Cyano, Aryl- oder Alkylsulfon, Aryl- oder Alkylsulfonyl} steht jeweils zwei benachbarte Reste
oder
R₁₋₅ zusammen für einem aromatischen, heteroaromatischen oder aliphatischen ankondensierten Ring stehen,
Alkyl für beliebige lineare, verzweigte oder cyclische Alkylreste mit 1 bis 40 C-Atomen steht, bei denen gegebenenfalls ein oder mehrere Wasserstoffatome durch Fremdatome oder Funktionen der Gruppe
{Fluor, gegebenenfalls Chlor oder Brom, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Fluor oder Chlor, Nitro, Cyano, Aryl- oder Alkylsulfon} substituiert sind,
R' und R" unabhängig voneinander für gleiche oder unterschiedliche Reste aus der Gruppe {Wasserstoff, Methyl, lineares, verzweigtes oder cyclisches, gegebenenfalls substituiertes Alkyl, gegebenenfalls substituiertes Phenyl} stehen
oder zusammen einen Ring bilden und für ein verbrückendes Strukturelement aus der Gruppe
{gegebenenfalls substituiertes Alkylen, verzweigtes Alkylen, cyclisches Alkylen oder gegebenenfalls substituiertes Azaalkylen} stehen,
wobei der Katalysator in Kombination mit einem oder mehreren phosphorhaltigen Liganden eingesetzt wird und der phosphorhaltige Ligand entweder
ein Ligand der Struktur 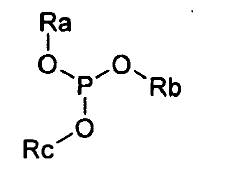 in Verbindung mit Palladium oder Nickel als Katalysator
ist, wobei
Ra, Rb und Rc unabhängig voneinander für jeweils einen geradkettigen, verzweigten oder cyclischen Alkylrest oder Arylrest steht, der gegebenenfalls Substituenten aus der Gruppe {geradkettiges oder verzweigtes Alkyl, Cycloalkyl, Aryl, Fluor, Chlor} trägt,
oder zwei dieser Reste zusammen einen Ring formen können und der Gruppe {gegebenenfalls substituiertes Alkylen, gegebenenfalls substituiertes ortho-Arylen} entstammen
oder drei dieser Reste einen Bicyclus formen können und der Gruppe {gegebenenfalls substituierte Trialkylole} entstammen
oder
ein Ligand der Struktur 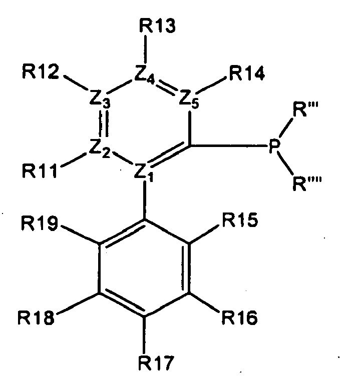 in Verbindung mit Palladium oder Nickel als Katalysator
ist, wobei
Z₁ für Kohlenstoff oder Stickstoff steht,
Z₂₋₅ unabhängig voneinander entweder für Kohlenstoff steht,
ZᵢRᵢ für Stickstoff steht, oder jeweils zwei benachbarte über eine formale Doppelbindung verbundene XᵢRᵢ mit i=2,3,4,5 gemeinsam für O (Furane), S (Thiophene), NH oder NRᵢ (Pyrrole) steht,
die Reste
R₁₁₋₁₉ für Substituenten aus der Gruppe
{Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl} steht
oder jeweils zwei benachbarte Reste
R₁₁₋₁₄ zusammen für einem aromatischen, heteroaromatischen oder aliphatischen ankondensierten Ring stehen,
R"' und R"" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe
{Alkylen, verzweigtes Alkylen, cyclisches Alkylen} oder unabhängig voneinander für einen oder zwei polycyclische Reste, wie z.B. Norbornyl- oder Adamantyl-, stehen,
oder
ein Komplex eines sekundären Phosphans in Verbindung mit einem Palladacyclus als Katalysator der Struktur 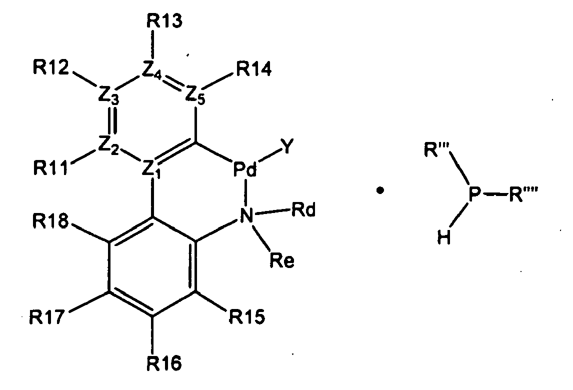 ist, wobei die Symbole
Z₁₋₅, R₁₁₋₁₈, R "' und R "" die vorstehend genannte Bedeutung haben und
Y für einen Rest aus der Gruppe {Halogenid, Pseudohalogenid, Alkylcarboxylat, Trifluoracetat, Nitrat, Nitrit} steht und
R_{d} und Rₑ unabhängig voneinander für gleiche oder unterschiedliche Substituenten aus der Gruppe
{Wasserstoff, Methyl, primäres, sekundäres oder tertiäres gegebenenfalls substituiertes Alkyl oder Aryl} stehen oder zusammen einen Ring bilden und aus der Gruppe {gegebenenfalls substituiertes Alkylen, Oxaalkylen, Thiaalkylen, Azaalkylen} entstammen.

2. Verfahren nach Anspruch 1, **dadurch gekennzeichnet dass** als Brønsted-Base ein Hydroxid, Amin, Alkoholat oder Fluorid der Alkali- oder Erdalkalimetalle, oder ein Alkalicarbonat, -hydrogencarbonat, -phosphat oder -monohydrogenphosphat oder Gemische dieser Verbindungen eingesetzt werden.

3. Verfahren nach Anspruch 2, **dadurch gekennzeichnet, dass** 1,0 bis 5 Äquivalente an Base bezogen auf die Boronsäure eingesetzt werden.

4. Verfahren nach Anspruch 1, **dadurch gekennzeichnet, dass** als Lösungsmittel Wasser, aliphatische Alkohole, aliphatische Glycole, aliphatische und/oder aromatische Ether, substituierte Aromaten, aliphatische Amide, Harnstoffe, Sulfoxide, N-Methylpyrrolidon oder ein Gemisch mehrerer dieser Lösungsmittel eingesetzt wird.

5. Verfahren nach mindestens einem der vorhergehenden Ansprüche, **dadurch gekennzeichnet, dass** die Reaktion bei einer Temperatur im Bereich von 20 bis 240°C durchgeführt wird.

6. Verfahren nach Anspruch 1, **dadurch gekennzeichnet, dass** der Katalysator im Verhältnis zum Reaktanden I in Mengen von 0.001 Mol-% bis 100 Mol% eingesetzt wird.

## Claims

1. A process for preparing alkyl-substituted aromatics
and heteroaromatics III by cross-coupling alkylboronic acids II with aryl or heteroaryl halides or aryl- or heteroarylsulfonates I, in the presence of a catalyst and of a Brønsted base, in a solvent or solvent mixture, 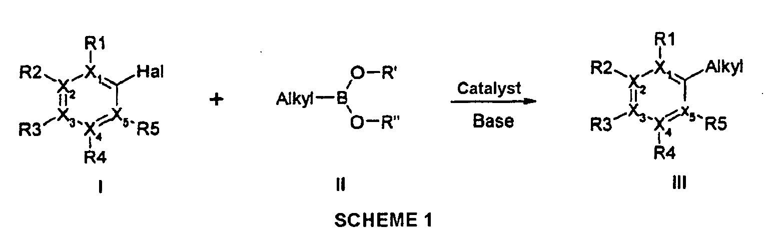 where
Hal is chlorine, bromine, iodine, trifluoromethanesulfonate, nonafluorotrimethylmethanesulfonate, methanesulfonate, 4-toluenesulfonate, benzenesulfonate, 2-naphthalenesulfonate, 3-nitrobenzenesulfonate, 4-nitrobenzenesulfonate, 4-chlorobenzenesulfonate or 2,4,6-triisopropylbenzenesulfonate,
X₁₋₅ are each independently carbon,
XᵢRᵢ is nitrogen, or in each case two adjacent XᵢRᵢ joined via a formal double bond together are O (furans), S (thiophenes), NH or NRᵢ (pyrroles),
the radicals
R₁₋₅ are substituents from the group of {hydrogen, methyl, primary, secondary or tertiary, cyclic or acyclic alkyl radicals having from 2 to 20 carbon atoms, in which one or more hydrogen atoms are optionally replaced by fluorine or chlorine or bromine, substituted cyclic or acyclic alkyl groups, hydroxyl, alkoxy, amino, alkylamino, dialkylamino, arylamino, diarylamino, alkylarylamino, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, fluorine or chlorine, nitro, cyano, aryl or alkyl sulfone, aryl- or alkylsulfonyl},
or in each case two adjacent radicals
R₁₋₅ together are an aromatic, heteroaromatic or aliphatic fused-on ring,
alkyl is any linear, branched or cyclic alkyl radicals having from 1 to 40 carbon atoms, in which one or more hydrogen atoms are optionally substituted by extraneous atoms or functions from the group of {fluorine, optionally chlorine or bromine, hydroxyl, alkoxy, amino, alkylamino, dialkylamino, arylamino, diarylamino, alkylarylamino, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, fluorine or chlorine, nitro, cyano, aryl or alkyl sulfone},
R' and R" are each independently identical or different radicals from the group of {hydrogen, methyl, linear, branched or cyclic alkyl, optionally substituted, phenyl, optionally substituted} or together form a ring and represent a bridging structural element from the group of {optionally substituted alkylene, branched alkylene, cyclic alkylene or optionally substituted azaalkylene},
the catalyst being used in combination with one or more phosphorus ligands and the phosphorus ligand being either a ligand of the structure 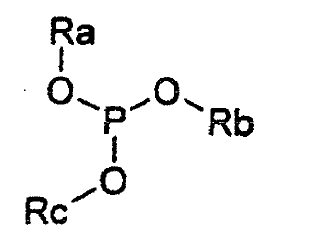 in conjunction with palladium or nickel as a catalyst, where
Ra, Rb and Rc are each independently a straight-chain, branched or cyclic alkyl radical or aryl radical which optionally bears substituents from the group of {straight-chain or branched alkyl, cycloalkyl, aryl, fluorine, chlorine}, or two of these radicals together may form a ring and stem from the group of {optionally substituted alkylene, optionally substituted ortho-arylene}, or three of these radicals may form a bicycle and stem from the group of {optionally substituted trialkylols}
or
a ligand of the structure 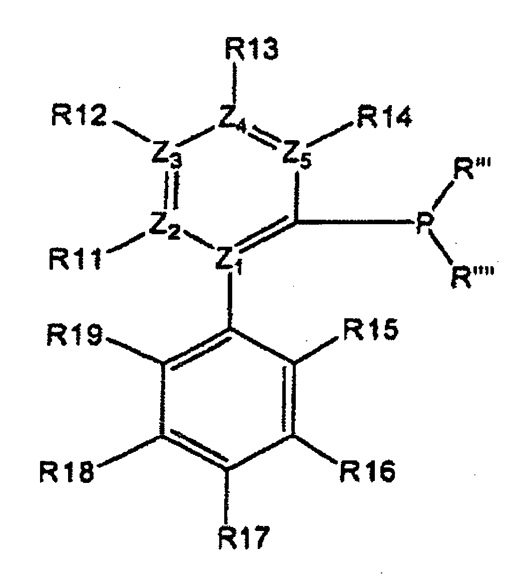 in conjunction with palladium or nickel as a catalyst, where
Z₁ is carbon or nitrogen,
Z₂₋₅ are each independently carbon,
ZᵢRᵢ is nitrogen or in each case two adjacent ZᵢRᵢ where i = 2, 3, 4, 5 joined via a formal double bond together are O (furans), S (thiophenes), NH or NRᵢ (pyrroles),
the R₁₁₋₁₉ radicals are substituents from the group of {hydrogen, methyl, primary, secondary or tertiary, acyclic or cyclic alkyl radicals having from 2 to 20 carbon atoms and in which one or more hydrogen atoms are optionally replaced by fluorine or chlorine or bromine, substituted cyclic or acyclic alkyl groups, hydroxyl, alkoxy, amino, alkylamino, dialkylamino, arylamino, diarylamino, alkylarylamino, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, nitro, cyano, aryl or alkyl sulfone, aryl- or alkylsulfonyl} or in each case two adjacent radicals
R₁₁₋₁₄ together are an aromatic, heteroaromatic or aliphatic fused-on ring,
R"' and R"" are each independently identical or different radicals from the group of {hydrogen, methyl, linear, branched or cyclic alkyl, phenyl} or together form a ring and are a bridging structural element from the group of {alkylene, branched alkylene, cyclic alkylene} or are each independently one or two polycyclic radicals, for example norbornyl or adamantyl,
or
a complex of a secondary phosphine in conjunction with a palladacycle as a catalyst of the structure 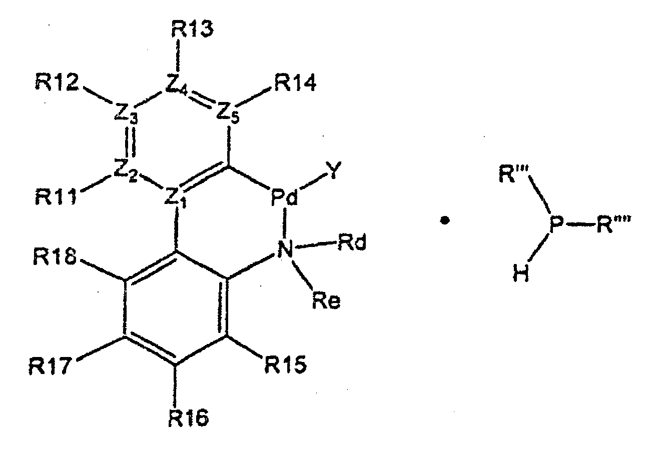 where the symbols
Z₁₋₅, R₁₁₋₁₈, R"' and R"" are each as defined above and
Y is a radical from the group of {halide, pseudohalide, alkyl carboxylate, trifluoroacetate, nitrate, nitrite} and
R_{d} and Rₑ are each independently identical or different substituents from the group of
{hydrogen, methyl, primary, secondary or tertiary, optionally substituted alkyl or aryl}
or together form a ring and stem from the group of {optionally substituted alkylene, oxaalkylene, thiaalkylene, azaalkylene}.

2. The process as claimed in claim 1, wherein the Brønsted base used is a hydroxide, amine, alkoxide or fluoride of the alkali metals or alkaline earth metals, or an alkali metal carbonate, hydrogencarbonate, phosphate or monohydrogenphosphate or mixtures of these compounds.

3. The process as claimed in claim 2, wherein from 1.0 to 5 equivalents of base based on the boronic acid are used.

4. The process as claimed in claim 1, wherein the solvents used are water, aliphatic alcohols, aliphatic glycols, aliphatic and/or aromatic ethers, substituted aromatics, aliphatic amides, ureas, sulfoxides, N-methylpyrrolidone or a mixture of a plurality of these solvents.

5. The process as claimed in at least one of the preceding claims, wherein the reaction is performed at a temperature in the range from 20 to 240 °C.

6. The process as claimed in claim 1, wherein the catalyst is used in amounts of from 0.001 mol% to 100 mol% in relation to the reactant I.

## Revendications

1. Procédé pour la préparation de composés aromatiques et hétéroaromatiques III substitués par alkyle, par combinaison par substitution d'acides alkylboroniques II avec des halogénures d'aryle ou d'hétéroaryle ou des sulfonates d'aryle ou d'hétéroaryle I, en présence d'un catalyseur et d'une base de Brönsted dans un solvant ou mélange de solvant 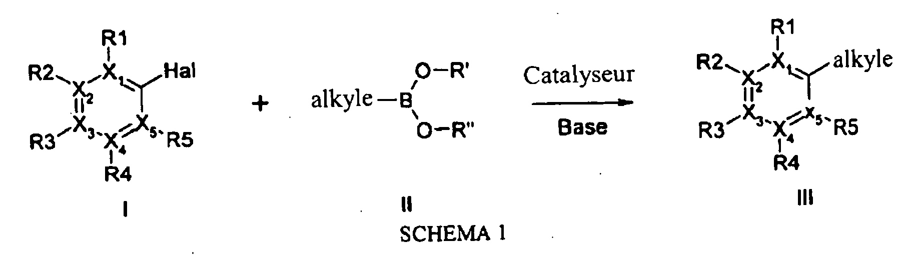 Où
Hal représente le chlore, le brome, l'iode, le groupe trifluorométhanesulfonate, nonafluorotriméthyl-méthanesulfonate, méthanesulfonate, 4-toluènesulfonate, benzènesulfonate, 2-naphtalènesulfonate, 3-nitrobenzènesulfonate, 4-nitrobenzènesulfonate, 4-chlorobenzènesulfonate ou 2,4,6-triisopropylbenzènesulfonate,
X₁₋₅ indépendamment les uns des autres, soit représentent un atome de carbone,
XᵢRᵢ représente un atome d'azote, soit respectivement deux XᵢRᵢ voisins, liés par une double liaison selon la formule, représentent ensemble O (furannes), S (thiophènes), NH ou NRᵢ (pyrroles),
les radicaux R₁₋₅ représentent des substituants choisis dans l'ensemble
{atome d'hydrogène, méthyle, radicaux alkyle primaires, secondaires ou tertiaires, cycliques ou acycliques, ayant de 2 à 20 atomes de carbone, dans lesquels éventuellement un ou plusieurs atomes d'hydrogène sont remplacés par le fluor ou le chlore ou le brome, groupes alkyle cycliques ou acycliques substitués, hydroxy, alcoxy, amino, alkylamino, dialkylamino, arylamino, diarylamino, alkylarylamino, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alcoxyalkyle, fluor ou chlore, nitro, cyano, aryl- ou alkylsulfone, aryl- ou alkylsulfonyle},
ou chaque fois deux radicaux voisins
R₁₋₅ forment ensemble un cycle aliphatique, aromatique ou hétéroaromatique condensé,
alkyle représente des radicaux alkyle linéaires, ramifiés ou cycliques quelconques, ayant de 1 à 40 atomes de carbone, dans lesquels éventuellement un ou plusieurs atomes d'hydrogène sont remplacés par des atomes étrangers ou des fonctions choisis dans l'ensemble
{fluor, éventuellement chlore ou brome, hydroxy, alcoxy, amino, alkylamino, dialkylamino, arylamino, diarylamino, alkylarylamino, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alcoxyalkyle, fluor ou chlore, nitro, cyano, aryl- ou alkylsulfone},
R' et R" représentent, indépendamment l'un de l'autre, des radicaux identiques ou différents, choisis dans l'ensemble
{hydrogène, méthyle, alkyle linéaire, ramifié ou cyclique, éventuellement substitué, phényle éventuellement substitué}
ou forment ensemble un cycle et représentent un élément structural pontant choisi dans l'ensemble
{alkylène éventuellement substitué, alkylène ramifié, alkylène cyclique ou aza-alkylène éventuellement substitué},
le catalyseur étant utilisé en association avec un ou plusieurs ligands phosphorés, et le ligand phosphoré
soit
est un ligand de formule 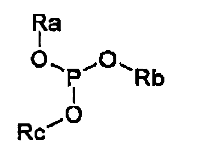 en association avec du palladium ou du nickel en tant que catalyseur
Ra, Rb et Rc représentant, chacun indépendamment, un radical alkyle à chaîne droite, ramifié ou cyclique ou un radical aryle, qui éventuellement porte des substituants choisis dans l'ensemble
{alkyle à chaîne droite ou ramifiée, cycloalkyle, aryle, fluor, chlore},
ou deux de ces radicaux pouvant former ensemble un cycle et étant choisis dans l'ensemble
{alkylène éventuellement substitué, ortho-arylène éventuellement substitué}
ou trois de ces radicaux pouvant former un cycle bicyclique et étant choisis dans l'ensemble
{trialkylols éventuellement substitués}
ou un ligand de formule 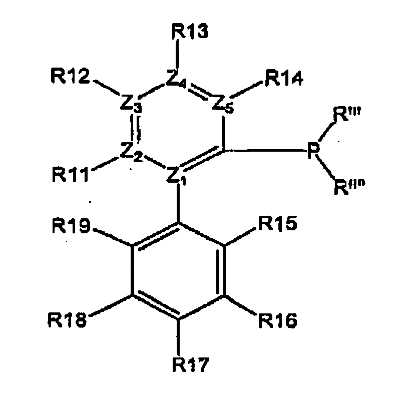 en association avec du palladium ou du nickel en tant que catalyseur, où
Z₁ représente un atome de carbone ou d'azote,
Z₂₋₅ indépendamment les uns des autres, soit représentent un atome de carbone,
ZᵢRᵢ représente un atome d'azote, soit respectivement deux XᵢRᵢ voisins, avec i = 2, 3, 4, 5, liés par une double liaison selon la formule, représentent ensemble O (furannes), S (thiophènes), NH ou NRᵢ (pyrroles),
les radicaux R₁₁₋₁₉ représentent des substituants choisis dans l'ensemble
{atome d'hydrogène, méthyle, radicaux alkyle primaires, secondaires ou tertiaires, cycliques ou acycliques, ayant de 2 à 20 atomes de carbone, dans lesquels éventuellement un ou plusieurs atomes d'hydrogène sont remplacés par le fluor ou le chlore ou le brome, groupes alkyle cycliques ou acycliques substitués, hydroxy, alcoxy, amino, alkylamino,
dialkylamino, arylamino, diarylamino, alkylarylamino, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alcoxyalkyle, nitro, cyano, aryl- ou alkylsulfone, aryl- ou alkylsulfonyle},
ou chaque fois deux radicaux voisins
R₁₁₋₁₄ forment ensemble un cycle aliphatique, aromatique ou hétéroaromatique condensé,
R"' et R"" représentent, indépendamment l'un de l'autre, des radicaux identiques ou différents, choisis dans l'ensemble
{hydrogène, méthyle, alkyle linéaire, ramifié ou cyclique, phényle} ou forment ensemble un cycle et représentent un élément structural pontant choisi dans l'ensemble
{alkylène, alkylène ramifié, alkylène cyclique},
ou représentent, indépendamment l'un de l'autre, un ou deux radicaux polycycliques, comme par exemple norbornyle ou adamantyle,
ou est un complexe d'un phosphane secondaire combiné à un cycle palladié en tant que catalyseur, de formule 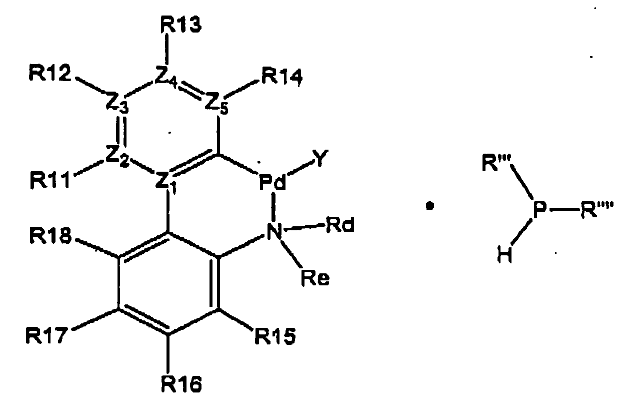 les symboles
Z₁₋₅, R₁₁₋₁₈, R"' et R"" ayant les significations données précédemment et
Y représentant un radical choisi dans l'ensemble {halogénure, pseudo-halogénure, alkylcarboxylate, trifluoroacétate, nitrate, nitrite} et
R_{d} et Rₑ représentant, indépendamment l'un de l'autre, des substituants identiques ou différents, choisis dans l'ensemble
{hydrogène, méthyle, alkyle primaire, secondaire ou tertiaire, éventuellement substitué ou aryle}
ou formant ensemble un cycle et étant choisis dans l'ensemble
{alkylène, oxa-alkylène, thia-alkylène, aza-alkylène éventuellement substitués}

2. Procédé selon la revendication 1, **caractérisé en ce qu'**on utilise comme base de Brönsted un hydroxyde, une amine, un alcoolate ou un fluorure des métaux alcalins ou alcalino-terreux, ou un carbonate, hydrogénocarbonate, phosphate ou monohydrogénophosphate de métal alcalin ou des mélanges de ces composés.

3. Procédé selon la revendication 2, **caractérisé en ce qu'**on utilise de 1,0 à 5 équivalents de base par rapport à l'acide boronique.

4. Procédé selon la revendication 1, **caractérisé en ce qu'**on utilise comme solvant l'eau, des alcools aliphatiques, des glycols aliphatiques, des éthers aliphatiques et/ou aromatiques, des composés aromatiques substitués, des amides aliphatiques, des urées, des sulfoxydes, la N-méthylpyrrolidone ou un mélange de plusieurs de ces solvants.

5. Procédé selon au moins l'une des revendications précédentes, **caractérisé en ce que** la réaction soit effectuée à une température dans la plage de 20 à 240 °C.

6. Procédé selon la revendication 1, **caractérisé en ce que** le catalyseur est utilisé en proportions allant de 0,001 % en moles à 100 % en moles, par rapport au partenaire réactionnel I.
